# EUROPEAN PATENT SPECIFICATION

(11) **EP 1 916 974 B1**
(45) Date of publication and mention of the grant of the patent: **07.09.2011**
(21) Application number: 06800119.7
(22) Date of filing: 19.07.2006
(51) Int. Cl.: A61F 7/12, A61M 5/44, A61F 9/00

(54) **OPHTHALMIC INJECTOR**
OPHTHALMISCHER INJEKTOR
INJECTEUR OPHTALMOLOGIQUE

(30) Priority: 22.08.2005 US 710046 P
(43) Date of publication of application: 07.05.2008
(73) Proprietor: Alcon Research, Ltd., Fort Worth TX 76134 (US)
(72) Inventor: MARSH, David A., Fort Worth, Texas 76179 (US); RODSTROM, Theron R., Cranfills Gap, Texas 76637 (US); WEINER, Alan L., Arlington, Texas 76001 (US)
(74) Representative: Hanna, Peter William Derek
(86) International application number: PCT/US2006/027955
(87) International publication number: WO 2007/024369

(56) References cited:
- DE-U1-202004 006 479
- US-A- 3 199 740
- US-A- 4 265 618
- US-A- 5 792 103
- US-A1- 2007 016 186
- US-A1- 2007 142 769
- US-B1- 6 488 659

## Description

### Field of the Invention

The present invention generally pertains to ophthalmic drug delivery and more particularly to posterior segment ophthalmic drug delivery.

### Description of the Related Art

Current intravitreal drug delivery devices deliver drugs to the vitreous through the pars plana region of the eye, which is devoid of retinal tissue, to avoid complications such as retinal detachment. Examples include the VITRASERT® implant and the RETISERT® implant available from Bausch & Lomb, both of which require a relatively large pars plana incision (e.g. 1-5 mm) for implantation. The OCUSERT device, which has been used to deliver pilocarpine for the treatment of glaucoma, employs a pars plana injection of biodegradable or bioerodible pellets through a 22 gauge needle, which is not "self-sealing" procedure.

U.S. Patent No. 4,030,499 to Bucalo discloses a syringe containing an implant in solid condition. The syringe includes a heating coil disposed on the exterior of or on the interior of the syringe barrel and the associated needle for converting the solid implant into a liquid prior to injection into the body tissue. However, such a coil design may result in increased manufacturing cost and potential reliability issues. U.S. Patent No. 6,488,659 to Rosenman discloses a catheter with heat transferring fluid passageways for injecting a thermally sensitive gelation material to remote sites within a patient's body. The thermally sensitive gelation material is delivered to the catheter via a syringe. The catheter maintains the thermally sensitive gelation material in a liquid state until it is delivered to the target issue. However, catheterized drug delivery is not suitable for ophthalmic applications.

US-4,265,618 describes an electrically heated endodontic syringe for injecting thermoplastic material, such as gutta percha, into a root canal cavity includes a syringe barrel for holding a supply of thermoplastic material. An electric resistance heating element carried by the heater body provides sufficient heat to maintain the entire needle above 230°F (110° C) at which the thermoplastic material will flow but below the temperature at which the material thermally decomposes.

Several diseases and conditions of the posterior segment of the eye continue to threaten vision. Age related macular degeneration (ARMD), choroidal neovascularization (CNV), retinopathies (e.g., diabetic retinopathy, vitreoretinopathy), retinitis (e.g., cytomegalovirus (CMV) retinitis), uveitis, macular edema, glaucoma, and neuropathies are several examples. Therefore, a need continues to exist for improved posterior segment ophthalmic drug delivery that does not suffer from the above-described limitations of current devices.

### Summary of the Invention

The present invention provides an ophthalmic injector according to the claims which follow.

The aspect of the present invention is an ophthalmic injector including a heating chamber, a dosage form, a needle, a plunger assembly, and a heater assembly. The dosage form is in a solid state and is disposed in the heating chamber. The needle is fluidly coupled to the heating chamber and has a length and a diameter enabling insertion into the vitreous at points other than the pars plana. The plunger assembly has a rod at least partially disposed in the heating chamber. The heater assembly is for heating the heating chamber to cause the dosage form to change from a solid state to a liquid state to enable the rod to inject the dosage form through the needle.

### Brief Description of the Drawings

For a more complete understanding of the present invention, and for further objects and advantages thereof, reference is made to the following description taken in conjunction with the accompanying drawings in which:
Figure 1 is a plan view of an ophthalmic injector according to a preferred embodiment of the present invention;
Figure 2 is a side, sectional view of the injector of Figure 1 taken along line 2-2; and
Figure 3 is an exploded, perspective view of the injector of Figure 1.

### Detailed Description of the Preferred Embodiments

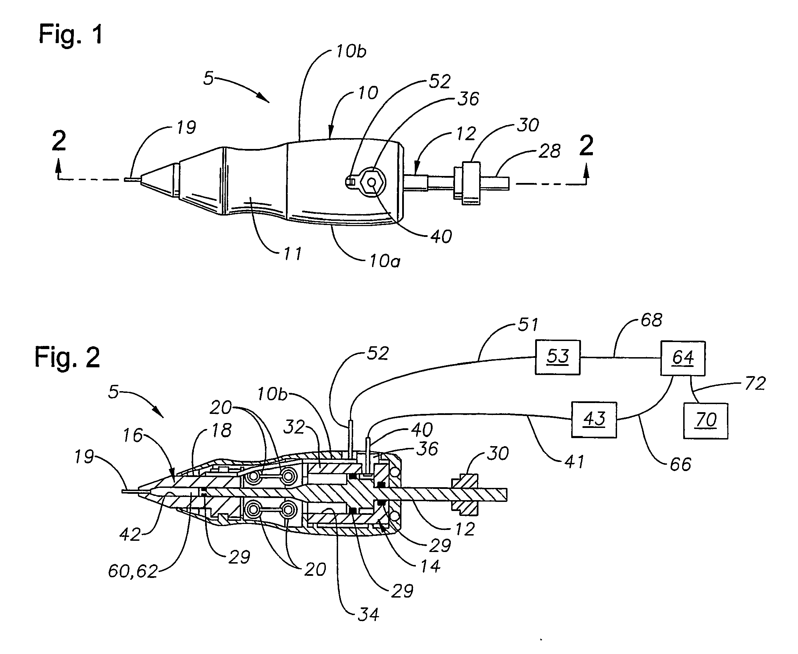

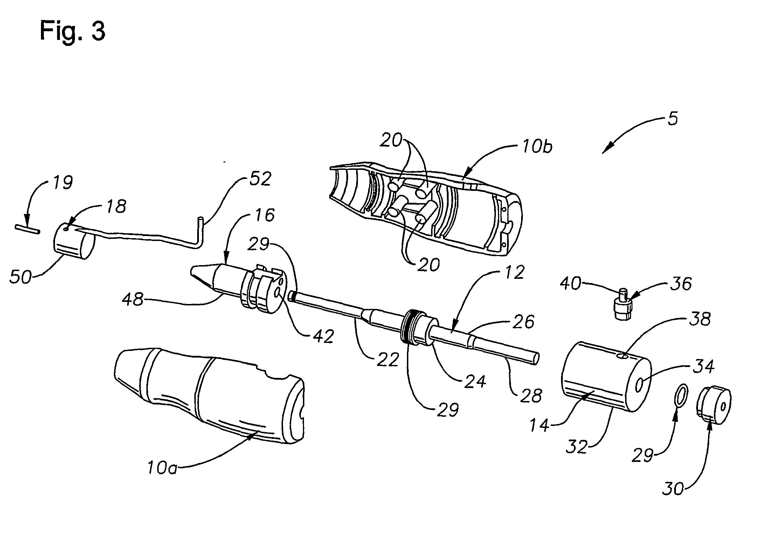

The preferred embodiments of the present invention and their advantages are best understood by referring to Figures 1-3 of the drawings, like numerals being used for like and corresponding parts of the various drawings.

Ophthalmic injector 5 generally includes a housing 10, a plunger assembly 12, an actuation assembly 14, a nose cone 16, a heater assembly 18, a needle 19, and a dosage form 60. Housing 10 includes a left housing 10a and a right housing 10b that are removably and frictionally coupled via pin mounts 20 and pins (not shown). Housing 10 preferably includes a region 11 for grasping ophthalmic injector 5. Plunger assembly 12 preferably includes a distal rod 22, a base 24, and a proximal rod 26. Rod 26 has a threaded end 28 for rotationally coupling with an adjustment nut 30. Plunger assembly 12 also includes o-rings 29 for fluidly sealing to various surfaces of ophthalmic injector 5. Actuation assembly 14 is preferably a cylinder 32 having a bore 34 for receiving plunger assembly 12 and for holding a volume of pressurized fluid. The pressurized fluid is preferably air. Cylinder 32 preferably has a fitting 36 for disposing within an opening 38. Fitting 36 has a lumen 40 in fluid communication with bore 34. Lumen 40 is fluidly coupled to tubing 41. Tubing 41 is for fluidly coupling with a source of pressurized fluid 43 external to ophthalmic injector 5. Nose cone 16 preferably includes a heating chamber 42 and a heater assembly mount 48. Nose cone 16 is preferably made from a material having a high thermal conductivity and is most preferably aluminum. Heater assembly 18 has a body 50 having a geometry for mating with heater assembly mount 48. Heater assembly 18 also has power leads 52 that are electrically coupled to an interface 51. Interface 51 is for electrically coupling to a power source 53 external to ophthalmic injector 5. Needle 19 is a self-sealing needle that is in fluid communication with heating chamber 42. Needle 19 is preferably a 23-25 gage, stainless steel needle having a length of about 0.5 to about 5 mm.

A dosage form 60 including a drug 62 is disposed in heating chamber 42 in a sterilized manner during the manufacture of ophthalmic injector 5. Dosage form 60 is a low melting point dosage form that is capable of delivery to the eye in a liquid state, solidifying at the target site, and delivering a controlled or sustained release amount of drug 62 to the target site. Dosage form 60 has a melting point between about 40 °C to about 80 °C and is biodegradable or bioerodible upon solidifying *in vivo.* Dosage form 60 is heated, disposed within heating chamber 42 in a liquid state, and then solidified. Heating chamber 42 preferably holds about 1 to about 100 µL of dosage form 60. Of course, the desired volume of dosage form 60 may vary for dosage forms with different drugs 62. Drug 62 may be any ophthalmically acceptable drug. Drug 62 is an ophthalmically acceptable drug for the treatment or prevention of a disease or condition of the posterior segment of the eye, including age related macular degeneration (ARMD), choroidal neovascularization (CNV), retinopathies (e.g., diabetic retinopathy, vitreoretinopathy), retinitis (e.g., cytomegalovirus (CMV) retinitis), uveitis, macular edema, glaucoma, and neuropathies. Dosage form 60 may also include other ophthalmically acceptable excipients, including excipients to alter its melting point.

Once dosage form 60 is disposed within heating chamber 42, left housing 10a and right housing 10b may be secured via pins and pin mounts 20. Adjustment nut 30 is then moved to the proper position along threaded end 28 of proximal rod 26 to adjust the throw of plunger assembly 12 for the proper volume of dosage form 60. Adjustment nut 30 is then preferably locked into place, preventing subsequent adjustment. Ophthalmic injector 5 may then be placed in its final packaging and sterilized.

Source of pressurized fluid 43 and power source 53 are preferably electrically coupled to a computer or microprocessor 64 via interfaces 66 and 68, respectively. A controller 70 is also preferably electrically coupled to microprocessor 64 via an interface 72. Controller 70 is preferably a foot controller.

A preferred method of using ophthalmic injector 5 to deliver a drug to the eye will now be described in greater detail. Ophthalmic injector 5 is particularly useful for drug delivery to the posterior segment of the eye. Because of the short length and self-sealing nature of needle 19, ophthalmic injector 5 can be inserted into the vitreous at locations other than the pars plana with a very low likelihood of disturbing the bulk of retinal tissue, even if needle 19 is inserted through the retina. The ability of ophthalmic injector 5 to inject a dosage form anywhere in the posterior segment maximizes drug concentration at the specific target tissue and decreases the potential for toxic side effects. In addition, ophthalmic injector 5 can be used to inject a dosage form to other portions of posterior segment or the anterior segment of the eye.

Prior to drug delivery, a nurse fluidly couples tubing 41 to source of pressurized fluid 43 and electrically couples interface 51 to power source 53. Within the sterile field, the physician activates power source 53 via controller 70. Heater assembly 18 heats nose cone 16, and thus heating chamber 42, so that dosage form 60 quickly transforms from a solid to a liquid state. Microprocessor 64 preferably customizes the amount of power and the heating time for a specific dosage form 60. The physician grasps ophthalmic injector 5 via region 11 and begins the delivery procedure on an anesthetized patient. Needle 19 is inserted into the vitreous of the eye at a portion of the posterior segment proximate the target tissue. Acutation assembly 14 is actuated via controller 70 to move distal rod 22 into heating chamber 42. Microprocessor 64 preferably controls the actuation pressure and time for a specific dosage form 60. Liquefied dosage form 60 flows from heating chamber 42, through needle 19, and into the target tissue. Once at the target tissue, dosage form 60 solidifies on the target tissue and begins controlled or sustained release delivery of drug 62. The physician removes needle 19 from the vitreous, and the opening caused by needle 19 self seals. Tubing 41 and interface 51 are uncoupled from power source 43 and source of pressurized fluid 53, respectively. Ophthalmic injector 5 is then preferably disposed of in a sharps collector.

Microprocessor 64 controls power source 53 so that heater assembly 18 heats dosage form 60 to a temperature where it remains in liquid form during its passage through needle 19 and until it reaches the target tissue but below a temperature where it could potentially damage or irritate the target tissue. Passive heat transfer between nose cone 16 and needle 19 preferably facilitates this process and eliminates the need to actively head needle 19 via an electric coil or other heating apparatus.

From the above, it may be appreciated that the present invention provides improved devices and methods for safe, effective, rate-controlled, localized delivery of a variety of drugs to the eye, and particularly to the posterior segment of the eye. The present invention is illustrated herein by example, and various modifications may be made by a person of ordinary skill in the art. For example, while the present invention is described above as using a plunger assembly 12 having a pneumatic actuation assembly 14, various other mechanical or electro-mechanical plunger and actuation assemblies may be utilized, such as a spring-actuated plunger assembly, an electrically powered linear actuator with plunger, or an electrically powered stepper motor with plunger.

It is believed that the operation and construction of the present invention will be apparent from the foregoing description. While the apparatus and methods shown or described above have been characterized as being preferred, various changes and modifications may be made therein without departing from the scope of the invention as defined in the following claims.

## Claims

1. An ophthalmic injector (5) for an eye, said eye having a pars plana, a vitreous and a retina comprising:
a heating chamber (42);
a dosage form (60) in a solid state disposed in said heating chamber;
a needle (19) fluidly coupled to said heating chamber*,* said needle having a length and a diameter enabling insertion into said vitreous other than said pars plana;
a plunger assembly (12) having a rod (22) at least partially disposed in said heating chamber; and
a heater assembly (18) for heating said heating chamber to cause said dosage form to change from a solid state to a liquid state to enable said rod to inject said dosage form through said needle,
**characterized in that** the dosage form has a melting point of from 40 to 80°C, is capable of delivery to the eye in a liquid state, resolidifying at a target site *in vivo,* and is biodegradable or bioerodable so as to deliver a controlled or sustained release amount of an ophthalmically acceptable drug (62) from the dosage form.

2. The ophthalmic injector of claim 1, further comprising:
a nose cone (16) having said heating chamber (42), and wherein said heater assembly (18) is disposed on said nose cone;
whereby said heater assembly is adapted to heat said nose cone and said heating chamber to cause said dosage form (60) to change from a solid state to a liquid state, and said nose cone is adapted to transfer sufficient heat to said needle (19) to prevent said dosage form in said liquid state from re-solidifying within said needle.

3. The ophthalmic injector of claim 2, wherein said heater assembly (18) does not actively heat said needle (19).

4. The injector of any one of claims 1 to 3, wherein said needle has a gauge of 23 or higher.

5. The injector of claim 4, wherein said needle has a gauge of 23 to 25.

6. The injector of claim 4 wherein said needle has a length of 0.5 mm to 5 mm.

7. The injector of any one of claims 1 to 6, wherein said needle enables insertion through the retina into the vitreous tissue of an eye, and/or through said retina into said vitreous without detachment of said retina.

8. The injector of any one of claims 1 to 7, wherein said needle is self-sealing.

## Patentansprüche

1. Ophthalmisches Injektionsgerät (5) für ein Auge, wobei das Auge eine Pars plana, einen Glaskörper und eine Netzhaut aufweist, umfassend:
eine Heizkammer (42);
eine in der Heizkammer angeordnete Dosierungsform (60) in einem festen Zustand;
eine durch ein Fluid mit der Heizkammer verbundene Nadel (19), wobei die Nadel eine Länge und einen Durchmesser besitzt, womit das Einsetzen in den Glaskörper außer der Pars plana ermöglicht wird;
eine Kolbeneinheit (12) mit einer zumindest teilweise in der Heizkammer angeordneten Stange (22); und
eine Heizkörpereinheit (18) zum Beheizen der Heizkammer, um eine Änderung der Dosierungsform von einem festen Zustand in einen flüssigen Zustand zu bewirken, damit die Stange die Dosierungsform durch die Nadel injizieren kann;
**dadurch gekennzeichnet, dass** die Dosierungsform einen Schmelzpunkt von 40 bis 80°C besitzt und geeignet ist, in einem flüssigen Zustand dem Auge zugeführt zu werden, an einer Zielstelle *in-vivo* wieder erstarrt und biologisch abbaubar oder biologisch auswaschbar ist, um eine gesteuerte oder anhaltende Freigabemenge eines ophthalmisch duldbaren Medikaments (62) von der Dosierungsform zuzuführen.

2. Ophthalmisches Injektionsgerät nach Anspruch 1, des Weiteren umfassend:
einen Nasenkegel (16), der die Heizkammer (42) aufweist und bei dem die Heizkörpereinheit (18) an dem Nasenkegel angeordnet ist;
wodurch die Heizkörpereinheit den Nasenkegel und die Heizkammer erwärmen kann, um zu bewirken, dass die Dosierungsform (60) sich von einem festen Zustand in einen flüssigen Zustand ändert und der Nasenkegel ausreichend Wärme an die Nadel (19) übertragen kann, um zu verhindern, dass die Dosierungsform in flüssigem Zustand innerhalb der Nadel wieder erstarrt.

3. Ophthalmisches Injektionsgerät nach Anspruch 2, bei dem die Heizkörpereinheit (18) die Nadel (19) nicht aktiv beheizt.

4. Injektionsgerät nach einem der Ansprüche 1 bis 3, bei dem die Nadel ein Normalmaß von 23 oder höher aufweist.

5. Injektionsgerät nach Anspruch 4, bei dem die Nadel ein Normalmaß von 23 bis 25 aufweist.

6. Injektionsgerät nach Anspruch 4, bei dem die Nadel eine Länge von 0,5 mm bis 5 mm aufweist.

7. Injektionsgerät nach einem der Ansprüche 1 bis 6, bei dem die Nadel das Einsetzen durch die Netzhaut in das Glaskörpergewebe eines Auges und/oder durch die Netzhaut in den Glaskörper ohne Ablösung der Netzhaut ermöglicht.

8. Injektionsgerät nach einem der Ansprüche 1 bis 7, bei dem die Nadel selbstabdichtend ist.

## Revendications

1. Injecteur ophtalmique (5) pour un oeil, ledit oeil présentant une pars plana, une humeur vitrée et une rétine, comprenant :
une chambre de chauffage (42) ;
une forme pharmaceutique (60) à l'état solide disposée dans ladite chambre de chauffage ;
une aiguille (19) couplée de manière fluidique à ladite chambre de chauffage, ladite aiguille présentant une longueur et un diamètre permettant une insertion dans ladite humeur vitrée ailleurs que par ladite pars plana ;
un ensemble formant piston (12) présentant une tige (22) au moins partiellement disposée dans ladite chambre de chauffage ; et
un ensemble formant corps de chauffe (18) pour chauffer ladite chambre de chauffage afin de provoquer le passage de ladite forme pharmaceutique d'un état solide à un état liquide afin de permettre à ladite tige d'injecter ladite forme pharmaceutique à travers ladite aiguille,
**caractérisé en ce que** la forme pharmaceutique présente un point de fusion allant de 40 à 80°C, est susceptible d'être administrée à l'oeil à l'état liquide, en se resolidifiant au niveau d'un site cible *in vivo,* et est biodégradable ou bioérodable de manière à administrer une quantité à libération contrôlée ou prolongée d'un médicament (62) ophtalmiquement acceptable à partir de la forme de dosage.

2. Injecteur ophtalmique selon la revendication 1, comprenant en outre :
une coiffe (16) présentant ladite chambre de chauffage (42), et ledit ensemble formant corps de chauffe (18) étant disposé sur ladite coiffe ;
ledit ensemble formant corps de chauffe étant adapté pour chauffer ladite coiffe et ladite chambre de chauffage afin de faire passer ladite forme pharmaceutique (60) d'un état solide à un état liquide, et ladite coiffe étant adaptée pour transférer suffisamment de chaleur vers ladite aiguille (19) pour empêcher ladite forme pharmaceutique dans ledit état liquide de se resolidifier dans ladite aiguille.

3. Injecteur ophtalmique selon la revendication 2, dans lequel ledit ensemble formant corps de chauffe (18) ne chauffe pas de manière active ladite aiguille (19).

4. Injecteur ophtalmique selon l'une quelconque des revendications 1 à 3, dans lequel ladite aiguille présente un calibre de 23 ou supérieur.

5. Injecteur ophtalmique selon la revendication 4, dans lequel ladite aiguille présente un calibre de 23 à 25.

6. Injecteur ophtalmique selon la revendication 4, dans lequel ladite aiguille présente une longueur de 0,5 mm à 5 mm.

7. Injecteur selon l'une quelconque des revendications 1 à 6, dans lequel ladite aiguille permet une insertion à travers la rétine dans le tissu vitré d'un oeil, et/ou à travers ladite rétine dans ladite humeur vitrée sans décoller ladite rétine.

8. Injecteur selon l'une quelconque des revendications 1 à 7, dans lequel ladite aiguille est auto-obturante.
